(19) Europäisches Patentamt
European Patent Office
Office européen des brevets

(11) Publication number: **0 116 963 A2**

(12) # EUROPEAN PATENT APPLICATION

(21) Application number: **84101620.7**

(22) Date of filing: **16.02.84**

(51) Int. Cl.³: **A 61 K 33/04**
//(A61K33/04, 31/55)

(30) Priority: **17.02.83 JP 25996/83**

(43) Date of publication of application:
**29.08.84 Bulletin 84/35**

(84) Designated Contracting States:
**BE DE FR GB NL SE**

(71) Applicant: **THE GREEN CROSS CORPORATION**
**15-1, Imabashi-1-chome Higashi-ku Osaka-shi**
**Osaka 541(JP)**

(72) Inventor: **Ueda, Yasuo**
**28-8-301, Shakusonjicho**
**Hirakata-shi(JP)**

(72) Inventor: **Kagitani, Yoshio**
**427-22, Yamanobocho**
**Kashihara-shi(JP)**

(72) Inventor: **Komeda, Shirou**
**1237, Nishikiori**
**Tondabayashi-shi(JP)**

(72) Inventor: **Funakoshi, Satoshi**
**3361, San Pasqual Street Pasadena**
**Los Angeles California 91107(US)**

(74) Representative: **Vossius Vossius Tauchner Heunemann Rauh**
**Siebertstrasse 4 P.O. Box 86 07 67**
**D-8000 München 86(DE)**

(54) **Injectable aqueous solution containing hydrogensulfite and/or sulfite and anticancerous benzodiazepine compound.**

(57) Described is an injectable aqueous solution containing a hydrogensulfite and/or sulfite and an anticancerous benzodiazepine compound of the formula I

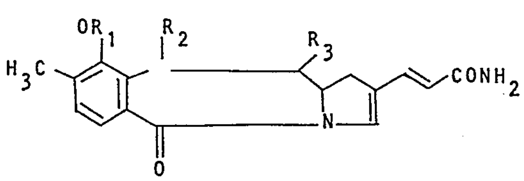

(I)

wherein $R_1$ denotes a hydrogen atom, or an acyl, carbamyl, or alkoxycarbonyl group; $R_2$ denotes a hydrogen atom or an acyl group; and $R_3$ denotes a sulfinic acid group, $SO_2X$, or a sulfonic acid group, $SO_3X$, wherein X denotes a hydrogen atom, an alkali metal or an alkaline earth metal, for controlling local injuries such inflammation and vein injuries occurring after injection.

EP 0 116 963 A2

Croydon Printing Company Ltd.

# INJECTABLE AQUEOUS SOLUTION CONTAINING HYDROGENSULFITE AND/OR SULFITE AND ANTICANCEROUS BENZODIAZEPINE COMPOUND

This invention relates to an injectable aqueous solution containing a hydrogen sulfin and an anticancerous benzodiazepine compound. This aqueous solution does not cause local-injuries after injection.

Nowadays, there are various compounds practically used as a medicine or proposed as such. For quite a few of these, however, an improvement is eagerly awaited, also many of these cannot yet be used. This is due to their property to cause local injuries for example when they are injected subcutaneously or when they leak out of the blood vessel after being injected intravenously cause an inflammation at the local region, or they cause injuries in the blood vessel when intravenously injected continually.

For example, some of the benzodiazepine compounds have been widely studied and developed as a carcinostatic or antibacterial agent. Also some of the present inventors have previously provided as a carcinostatic agent 1H-pyrrolo [2,1-c] [1,4] benzodiazepin-2-acrylamide compounds represented by the general formula I

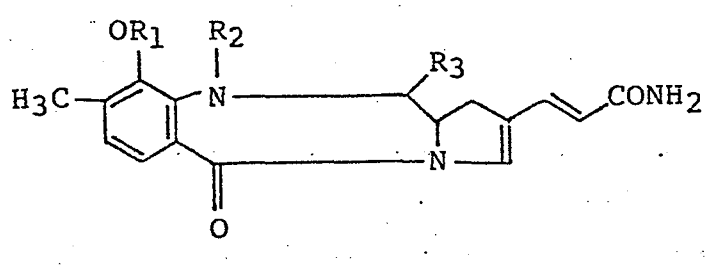

(I)

1

wherein $R_1$ denotes a hydrogen atom or an acyl, carbamyl or alkoxycarbonyl group; $R_2$ denotes a hydrogen atom or an acyl group, and $R_3$ denotes a sulfinic acid group, $SO_2X$, or a sulfonic acid group, $SO_3X$, wherein X denotes a hydrogen atom, an alkali metal such as sodium and potassium, or an alkaline earth metal such as calcium [Japanese Patent Application "Kokai" (Laid-open) No. 15289/81, U.S. Pat. No. 4309437 and German Patent No. 3,010,544 ].

On subsequent studies, however, it was confirmed that these benzodiazepine compounds, when leaked out of the blood vessel on administration to a living body, caused a serious inflammation resulting in necrosis.

Thus, the object of this invention is to provide a medicine capable of controlling the local injuries as mentioned above. The invention has been accomplished based on the following knowledge.

Namely, the inventors have found that, when a compound having a property to cause a local injury, such as the benzodiazepine compounds mentioned above, is administered jointly with a hydrogensulfite and/or a sulfite, local injuries occuring after parenteral or intraperitoneal administration and also local injuries of blood vessels occurring on continous intravenous administration are prevented.

According to the invention, there is provided an injectable aqueous solution containing a physiologically acceptable metal hydrogensulfite and/or sulfite and an effective amount for controlling tumor of a benzodiazepine compound of the general formula I

1

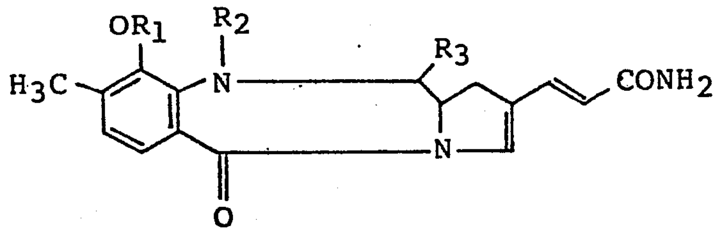

[I]

wherein $R_1$ denotes a hydrogen atom, or an acyl, carbamyl or alkoxycarbonyl group; $R_2$ denotes a hydrogen atom or an acyl group; $R_3$ denotes a sulfinic acid group, $SO_2X$, or a sulfonic acid group, $SO_3X$, wherein X denotes a hydrogen atom, an alkali metal or an alkaline earth metal, the amount of the hydrogensulfite and/or sulfite being 5-15 times the amount of the benzodiazepine compound, i.e. the weight ratio is from 5:1 to 15:1.

Preferred examples of physiologically acceptable metal salts of the hydrogensulfite and sulfite used in this invention include alkali metal salts such as the sodium or potassium salt. Of these, particularly preferred are sodium hydrogensulfite and sodium sulfite.

The term acyl denotes a benzoyl or an alkanoyl group having 1 to 6 carbon atoms; the carbamyl group may have its nitrogen atom substituted with phenyl group or an alkyl group containing 1 to 6 carbon atoms. The alkoxycarbonyl group contains 1 to 6 carbon atoms in the alkoxy group.

Specific examples of the benzodiazepine compounds used in the invention are:

5,10,11,11a-Tetrahydro-10-acetyl-9-hydroxy-8-methyl-5-oxo-11-sulfo-1H-pyrrolo[2,1-c][1,4]benzodiazepin-2-acrylamine and alkali metal salts or alkaline eath metal salts thereof;

5,10,11,11a-Tetrahydro-9-acetoxy-10-acetyl-8-methyl-5-oxo-11-sulfo-1H-pyrrolo[2,1-c][1,4]benzodiazepin-2-acrylamide and alkali metal salts or alkaline earth metal salts thereof;

5,10,11,11a-Tetrahydro-10-acetyl-9-carbamyloxy-8-methyl-5-oxo-11-sulfo-1H-pyrrolo[2,1-c][1,4]benzodiazepin-2-acrylamide and alkali metal salts or alkaline earth metal salts thereof;

5,10,11,11a-Tetrahydro-9-hydroxy-8-methyl-5-oxo-11-sulfo-1H-pyrrolo[2,1-c][1,4]benzodiazepin-2-acrylamide and alkali metal salts or alkaline earth metal salts thereof;

5,10,11,11a-Tetrahydro-9-acetoxy-8-methyl-5-oxo-11-sulfo-1H-pyrrolo[2,1-c][1,4]benzodiazepin-2-acrylamide and alkali metal salts or alkaline earth metal salts thereof;

5,10,11,11a-Tetrahydro-9carbamyloxy-8-methyl-5-oxo-11-sulfo-1H-pyrrolo[2,1-c][1,4]benzodiazepin-2-acrylamide and alkali metal salts or alkaline earth metal salts thereof;

5,10,11,11a-Tetrahydro-10-acetyl-9-hydroxy-8-methyl-5-oxo-11-sulfino-1H-pyrolo[2,1-c][1,4]benzodiazepin-2-acrylamide and alkali metal salts or alkaline earth metal salts thereof;

5,10,11,11a-Tetrahydro-9-acetoxy-10-acetyl-8-methyl-5-oxo-11-sulfino-1H-pyrrolo[2,1-c][1,4]benzodiazepin-2-acrylamide and alkali metal salts or alkaline earth metal salts thereof;

5,10,11,11a-Tetrahydro-10-acetyl-9-carbamyl-8-methyl-5-oxo-11-sulfino-1H-pyrrolo[2,1-c][1,4]benzodiazepin-2-acrylamide and alkali metal salts or alkaline earth metal salts thereof;

5,10,11,11a-Tetrahydro-9-hydroxy-8-methyl-5-oxo-11-sulfino-1H-pyrrolo[2,1-c][1,4]benzodiazepin-2-acrylamide and alkali metal salts or alkaline metal salts thereof;

5,10,11,11a-Tetrahydro-9-carbamyloxy-8-methyl-5-oxo-11-sulfino-1H-pyrrolo[2,1-c][1,4]benzodiazepin-2-acrylamide and alkali metal salts or alkaline earth metal salts thereof,

5,10,11,11a-Tetrahydro-8-methyl-9-methoxycarbonyloxy-5-oxo-11-sulfo-1H-pyrrolo[2,1-c][1,4]-benzodiazepin-2-acrylamide and alkali metal salts or alkaline earth metal salts thereof.

Their effective amount for controlling tumors in test animals is disclosed in U.S. Patent No. 4,309,437 and others. For treating human tumors the dose is in the range of 0.01-0.5 mg/Kg/day.

Thus, the benzodiazepine compound is administered parenterally as an injectable aqueous solution containing 1 to 5000 mg/l for a patient.

The injectable solution of the present invention is prepared preferably at the time of use by dissolving the two components in distilled water or the like. The compounds may, thus, preferably be in the form of a kit of the combination of the dry separate components which may be dissolved at the time of use. For making the solution it is advantageous to dissolve the hydrogensulfite or the sulfite at first and then the benzodiazepine compound in the solution.

The injectable aqueous solution of the invention can be administered parenterally, for example intravenously. The dosage of the hydrogen sulfite or sulfite may be generally 5 to 15 times, preferably 5 to 10 times, the effective one of the benzodiazepine compound. For instance, when 2 mg of a benzodiazepine compound is to be administered, it is preferably used after having been dissolved in 5 ml of a physiological saline solution containing 20 mg of the hydrogensulfite or sulfite.

Example 1

Four injectable aqueous solutions of the invention were prepared by dissolving sodium hydrogensulfite or sodium sulfite and then the sodium salt of 5,10,11,11a-terahydro-9-hydroxy-8-methyl-5-oxo-11-sulfino-1H-pyrrolo[2,1-c][1,4]benzodiazepin-2-acrylamide

(herein referred to simply as "Compound B") in a physiological saline solution. The concentrations of each component are shown in Table 1 (solution No. 2, 3, 4 and 5).

On the other hand, there are prepared injectable aqueous solutions containing Compound B, sodium sulfite and sodium hydrogen sulfite alone, respectively, in each concentration indicated in Table 1 (solution No. 1, 6 and 7).

Using the solutions together with the physiological saline solution serving as a control (No. 8), tests for observing tissue injury occuring when the compound B leaked out of the blood vessel were conducted by subcutaneously injecting 0.1 ml each of the solutions once in the neighborhood of the ear vein of white rabbits (male, 2.0-2.3 Kg). The change of ear thickness (formation of edema) in each of the treated groups was examined for one week after administration.

Table 1

| Solution No. | Compound B mg/ml | $NaHSO_3$ mg/ml | $Na_2SO_3$ mg/ml |
|---|---|---|---|
| 1 | 0.2 | 0 | 0 |
| 2 | 0.2 | 1.0 | 0 |
| 3 | 0.2 | 0 | 1.0 |
| 4 | 0.2 | 2.0 | 0 |
| 5 | 0.2 | 0 | 2.0 |
| 6 | 0 | 2.0 | 0 |
| 7 | 0 | 0 | 2.0 |
| 8 | 0 | 0 | 0 |

Figure 1 is a graph showing the local-injury controlling effect (the change of ear thickness of the rabbit) in combined administration to the ear of the rabbit of the compound B with the sulfite or hydrogen-sulfite. Curves 1 to 7 show respectively the effects in the administration of the preparations corresponding to the solutions No. 1 to 7, and the curve 8 is that for the physiological saline.

The results revealed, as can be seen in Fig. 1, a statistically significant tissue-injuring controlling effect obtained by the use of $NaHSO_3$ and/or $Na_2SO_3$. In the figure, the ratio of change of the ear thickness was expressed by the value of ratio of the increase of ear thickness after administration to

the thickness just before administration at the administered site (edema ratio)

Edema ratio % =

$$\frac{\text{Ear thickness after administration} - \text{Ear thickness just before administration}}{\text{Ear thickness just before administration}} \times 100$$

Example 2

The same materials as those in Examle 1 were used to determine the effect thereof administered to white rabbits (male, 2.0-2.3 kg) in groups of four in the ear vein once a day for 5 consecutive days. The material administered to the respective groups was (1) physiological saline, or (2) 0.16 mg of the compound B/Kg/day, (3) 0.16 mg of the compound B + 1.6 mg of $NaHSO_3$/Kg/day or (4) 1.6 mg of $NaHSO_3$/Kg/day. The solution volumes to be administered were all fixed at 0.5 ml/Kg/day containing the respective material or materials in the above dosage.

For confirmation of the effect, the appearance was observed and the thickness at the administration site was measured 4 days after the final administration to serve as the index of tissue injury.

The edema ratio was determined according to the following equation.

Edema ratio % =

$$\frac{\text{Thickness at administration site} - (A)}{\text{Thickness of ear on opposite side of medicine administration (A)}} \times 100$$

Table 2

| | Edema ratio (%) |
|---|---|
| 1 | 5.4 |
| 2 | 169.4 |
| 3 | 28.3 |
| 4 | 0.0 |

The results confirmed that the combined use of the benzodiazepine compound with $NaHSO_3$ exhibits a statistically significant controlling effect.

Example 3

It was confirmed that the stability of the compound B was not affected when an aqueous solution of $NaHSO_3$ or $Na_2SO_3$ was used as solvent for the compound B. In a sample of 10 ml of an aqueous solution containing 0.2 mg of the compound B and 2.0 mg of $NaHSO_3$ or $Na_2SO_3$ in 1 ml thereof, analysis was made by high-performance liquid chromatography just after dissolving the compound B and after 3 hours standing at room temperature, to examine the stability of the compound B. The results are as shown in Table 3.

Table 3

| | Ratio of remaining compound B |
|---|---|
| Compound B alone | 100% |
| Sample | 100% |

Example 4

For confirming the antitumor activity of the injectable aqueous solution of this invention P 388 leukemia cancer-bearing $BDF_1$ mice (male, 20-22 g) were used in groups of four. An aqueous solution containing 0.2 mg of the compound B + 2.0 mg of sodium hydrogensulfite or sodium sulfite in 1 ml thereof was administered intravenously once a day, for 4 consecutive days. The dosage is indicated in Table 4.

By the comparative examination of mean survival days of the mice, the index of life saving was calculated according to the following equation:

Index of life saving % =

$$\frac{\text{mean survival days of the administered group} - (B)}{\text{mean survival days of the control group (B)}} \times 100$$

The results are as shown in Table 4.

Table 4

| Administration group of material | Mean survival days | Index of life saving (%) |
|---|---|---|
| Physiological saline (control) | 10 | 0 |
| Compound B 2 mg/Kg/day | 18.0 | 80 |
| Compound B 2 mg/Kg/day $NaHSO_3$ 20 mg/kg/day | 17.0 | 70 |
| Compound B 2 mg/Kg/day $Na_2SO_3$ 20 mg/Kg/day | 17.0 | 70 |

The results showed that the antitumor activity of the compound B was not affected at all by the simultaneous administration of 10 times its amount of $NaHSO_3$ or $Na_2SO_3$.

Example 5

The materials were administered to ddY strain mice (male, 18-20 g) intravenously to determine the $LD_{50}$ (mg/Kg). The results showed that the $LD_{50}$ of the group to which the compound B alone was administered was 6.23, while that of the group to which the compound B and 10 times its amount of $NaHSO_3$ were administered in combination was 6.17, confirming that $NaHSO_3$ did not affect the acute toxicity of the compound B at all.

Incidentally, $NaHSO_3$ or $Na_2SO_3$ itself is a substance prescribed in Japanese Parmacopoeia and its safety has been established already.

What is claimed is

1. An injectable aqueous solution containing a physiologically acceptable metal hydrogensulfite and/or sulfite and an effective amount for controlling tumor of a benzodiazepine compound of the formula I

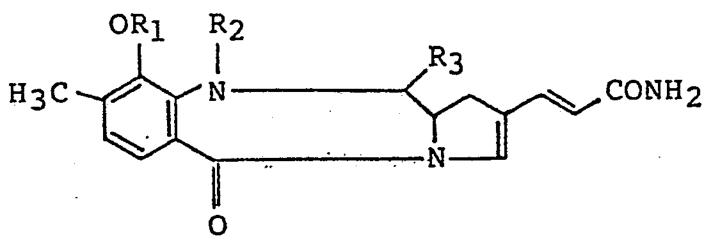

(I)

wherein $R_1$ denotes a hydrogen atom, or an acyl, carbamyl or alkoxycarbonyl group; $R_2$ denotes a hydrogen atom or an acyl group; and $R_3$ denotes a sulfinic acid group, $SO_2X$, or a sulfonic acid group, $SO_3X$, wherein X denotes a hydrogen atom, an alkali metal or an alkaline earth metal, the amount of the hydrogensulfite and/or sulfite being 5-15 times the amount of the benzodiazepine compound.

2. The solution of Claim 1, wherein the benzodiazepine compound is 5,10,11,11a-tetrahydro-9-hydroxy-8-methyl-5-oxo-11-sulfino-1H-pyrrolo[2,1-c]-[1,4]benzodiazepin-2-acrylamide.

3. The solution of Claim 1, wherein the effective amount of the benzodiazepine compound is 1 to 5000 mg/l.

4. The solution of Claim 1, wherein the physiologically acceptable metal is an alkali metal.

5. The solution of Claim 4, wherein the alkali metal is sodium.

6. A pharmaceutical kit of a combination of alkali metal hydrogensulfite and/or sulfite and a benzodiazepine compound of the formula I

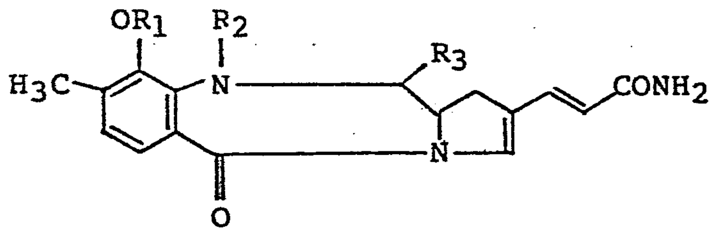

(I)

wherein $R_1$ denotes a hydrogen atom, or an acyl, carbamyl, or alkoxycarbonyl group; $R_2$ denotes a hydrogen atom or an acyl group; and $R_3$ denotes a sulfinic acid group, $SO_2X$, or a sulfonic acid group, $SO_3X$, wherein X denotes a hydrogen atom, an alkali metal or an alkaline earth metal.

7. The use of the injectable aqueous solution according to claim 1 for controlling malignant neoplasias and the occurence of local injuries on injection.

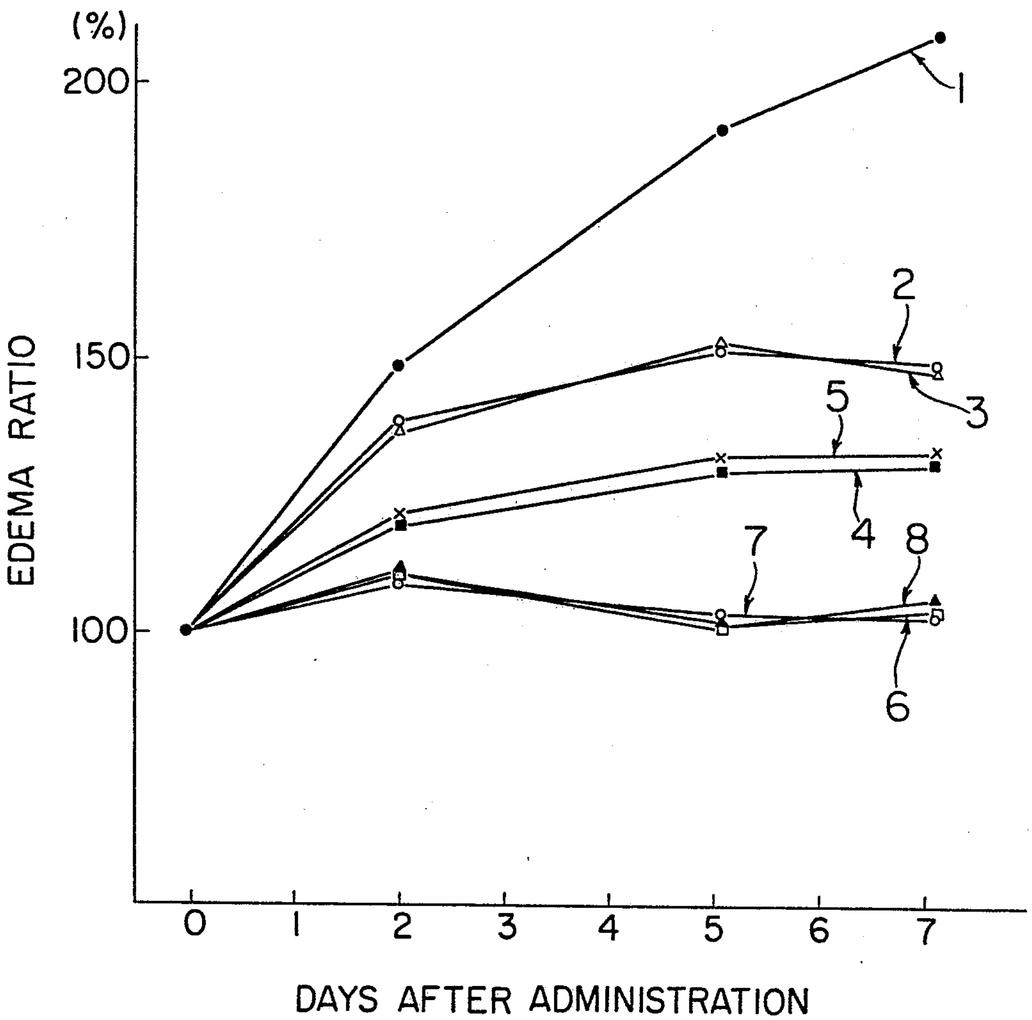
(%)
200
150
100
EDEMA RATIO
0
1
2
3
4
5
6
7
DAYS AFTER ADMINISTRATION
1
2
3
4
5
6
7
8